# EUROPEAN PATENT SPECIFICATION

(11) **EP 2 722 673 B1**
(45) Date of publication and mention of the grant of the patent: **12.07.2017**
(21) Application number: 12188904.2
(22) Date of filing: 17.10.2012
(51) Int. Cl.: G01N 33/68

(54) **Improved method of mapping glycans of glycoproteins**
Verbessertes Verfahren zum Mapping von Glycanen von Glycoproteinen
Procédé amélioré de mise en correspondance de glycanes de glycoprotéines

(43) Date of publication of application: 23.04.2014
(73) Proprietor: Hexal AG, 83607 Holzkirchen (DE)
(72) Inventor: Higel, Fabian, D-82041 Oberhaching (DE); Seidl, Andreas, D-82041 Oberhaching (DE)
(74) Representative: Dörries, Hans Ulrich

(56) References cited:
- WO-A1-2006/114663
- BIGGE J C ET AL: "Nonselective and efficient fluorescent labelling of glycans using 2-amino benzamide and anthranilic acid", ANALYTICAL BIOCHEMISTRY, ACADEMIC PRESS INC, NEW YORK, vol. 230, no. 2, 1 January 1995 (1995-01-01), pages 229-238, XP002489980, New York NY USA ISSN: 0003-2697, DOI: 10.1006/ABIO.1995.1468
- Sigma: "Technical Bulletin: GlycoProfile 2-AA Labeling kit", 1 April 2005 (2005-04-01), Sigmq-Aldrich Inc., St louis MI USA, XP055055920, pages 1-4, * page 3, section "Analysis of 2-AA labeled glycans"; page 4, section "references", nrs 1-8 *

## Description

### Field of the Invention

The present invention relates to the determination of glycosylation patterns in glycoproteins, e.g., in antibodies. Specifically, the present invention relates to methods of labeling neutral or acidic glycans of glycoproteins, such as antibodies, with anthranilic acid (2-AA) and separating the labeled glycans using reversed-phase liquid chromatography (RP-LC), followed by positive ionization mass spectrometry (MS). The invention also relates to the use of anthranilic acid (2-AA) in the method of the invention. The present invention avoids the need for an ion-pairing reagent and is especially useful for the separation of neutral and acidic N-glycans of glycoproteins.

### Background of the Invention

Biological drugs belong to the most complex pharmaceuticals. One important class of biological drugs are glycoproteins, and in particular monoclonal antibodies (mAbs), which are typically glycoproteins with a molecular mass of about 150 kDa. In recent years the glycosylation of therapeutic proteins came into focus as several investigations showed that these structures may have an effect on the safety and efficacy of the corresponding biological drugs. IgGs have one conserved N-glycosylation site on each heavy chain at their Fc part, usually around amino acid 297. The heterogeneity of the N-glycans attached to these sites is very high and more than a dozen different glycans can be found. Identification and quantification of glycans from this mixture is sophisticated and demands a comprehensive analytical approach. Structural isomers of several glycans make the discrimination even more difficult.

N-glycosylation is a critical issue for the safety and efficacy of biopharmaceuticals. Hence, comprehensive analysis of N-glycosylation is necessary. N-glycosylation can be studied after enzymatic release of the glycans by PNGaseF using liquid chromatography, mass spectrometry or by a combination of both technologies (Chen, X. & Flynn, G.C. Analysis of N-glycans from recombinant immunoglobulin G by on-line reversed-phase high-performance liquid chromatography/mass spectrometry. Analytical Biochemistry 370, 147-61 (2007)). Structural analysis of underivatized or labeled N-glycans is usually done by matrix-assisted laser desorption/ionization (MALDI-MS) or by porous graphitized carbon (PGC) liquid chromatography and on-line electrospray ionization mass spectrometry (ESI MS/MS).

Underivatized N-glycans have no light absorbing properties, so information gained from this kind of analytical methods is limited to mass spectrometry (MS) data. To circumvent these limitations, the N-glycans are often derivatized with fluorescence labels. For quantitative analytics of N-glycans, labeling with a fluorescence dye through reductive amination is widely used, offering high sensitivity. Every N-glycan carries only one label independent of size or branching, therefore, quantitative information can be obtained from the fluorescence signal intensity. The tag can not only provide UV or fluorescence detection but also the efficiency in electrospray ionization can be improved. For the analysis of sialic acid-containing glycans, labeling with a negatively charged tag, like 2-aminobenzoic acid (2-AA) or 8-aminonaphthalene-1,3,6-trisulfonic acid (ANTS), and MS detection in negative ionization mode is frequently used (Prien, J.M., Prater, B.D., Qin, Q. & Cockrill, S.L. Mass spectrometric-based stable isotopic 2-aminobenzoic acid glycan mapping for rapid glycan screening of biotherapeutics. Analytical Chemistry 82, 1498-508 (2010)).

From the multitude of available fluorescence tags, 2-aminobenzamide (2-AB) is very popular and frequently used. A widely applied method is the separation and quantitation of 2-AB labeled N-glycans on a normal phase HILIC (Hydrophilic Interaction Liquid Chromatography) column. HILIC offers very good resolution and separation of many glycan isomers. However, the small injection volume of aqueous samples leads to a lowered sensitivity. Therefore, alternative approaches were developed like the use of reversed phase chromatography together with ESI-MS (Electrospray Ionization-Mass Spectrometry).

Bigge JC, et al., 1995 (Analytical Biochemistry. Academic Press Inc., New York; 230(2):229-238) discloses reaction conditions for the conjugation of 2-AB and 2-AA to N- and O-glycans and the Sigma "Technical Bulletin: GlycoProfile™ 2-AA Labeling kit" (1 April 2005, Sigma-Aldrich Inc., St. Louis, MI, USA) describes the GlycoProfile™ 2-AA Labeling kit and how to use it for labeling glycans.

Since RP mobile phases usually only consist of water, an organic solvent and an acid, they are highly MS compatible. In addition there are almost no limitations with respect to injection volumes offering high sensitivity. It has been shown that RP-LC-MS of 2-AB, 2-AA or ANTS derivatized oligosaccharides can be performed (Chen, X. & Flynn, G.C. Analysis of N-glycans from recombinant immunoglobulin G by on-line reversed-phase high-performance liquid chromatography/mass spectrometry. Analytical biochemistry 370, 147-61 (2007); Prien, J.M., Prater, B.D., Qin, Q. & Cockrill, S.L. Mass spectrometric-based stable isotopic 2-aminobenzoic acid glycan mapping for rapid glycan screening of biotherapeutics. Analytical Chemistry 82, 1498-508 (2010); Prater, B.D., Connelly, H.M., Qin, Q. & Cockrill, S.L. High-throughput immunoglobulin G N-glycan characterization using rapid resolution reverse-phase chromatography tandem mass spectrometry. Analytical Biochemistry 385, 69-79 (2009)). For example, RP-HPLC (reversed-phase high performance liquid chromatography) coupled with ESI-MS has been used for the analysis of 2-AB-labeled N-glycans, including those released from a recombinant IgG antibody (Chen, X. & Flynn, G.C. Analysis of N-glycans from recombinant immunoglobulin G by on-line reversed-phase high-performance liquid chromatography/mass spectrometry. Analytical Biochemistry 370, 147-61 (2007)). However, the method is not very selective for isomers and is time consuming. If 2-AB is used as fluorescence tag, only weak hydrophobicity is provided to the hydrophilic glycan, which results in a shallow and long chromatography gradient. Labeled acidic glycans elute early and are poorly separated. This problem was addressed in the art by the addition of an ion pairing reagent which increases the pH and thereby lowers the ionization efficiency (Melmer, M., Stangler, T., Premstaller, A. & Lindner, W. Comparison of hydrophilic-interaction, reversed-phase and porous graphitic carbon chromatography for glycan analysis. Journal of Chromatography. A 1218, 118-23 (2011)).

Negatively charged labeling, in combination with negative ionization in MS, has been frequently used to analyze acidic N-glycans (Prien, J.M., Prater, B.D. & Cockrill, S.L. A multi-method approach toward de novo glycan characterization: a Man-5 case study. Glycobiology 20, 629-47 (2010)). For the characterization of oligomannose structures, 2-AA derivatized N-glycans were analyzed by a combination of reversed phase chromatography and negative mode mass spectrometry (Prien, J.M., Prater, B.D. & Cockrill, S.L. A multi-method approach toward de novo glycan characterization: a Man-5 case study. Glycobiology 20, 629-47 (2010)). According to this approach, N-glycans labeled with 2-AA can be separated using resolution reversed-phase separation with acetic acid-based mobile phases. However, separation of other isomers like G1F isomers with 1,3 and 1,6 galactosylation, which are highly abundant in most mAbs, has not previously been shown in any N-glycan reversed phase chromatography approach.

Also, prior to the present invention, an efficient and sensitive analysis of neutral and acidic N-glycans by RP-LC-MS in one approach was not available, since acidic 2-AB N-glycans elute in broad peaks at the beginning of the chromatographic run. Previous methods have been mostly limited to the isomer separation of oligomannose type N-glycans.

The inventors have now surprisingly found that the use of 2-AA for labeling glycoprotein glycans may overcome these disadvantages. Specifically, the inventors have found that 2-AA labeling of neutral or acidic glycoprotein glycans is advantageous where the glycans are thereafter separated via RP-LC using an acidic mobile phase and subsequently analyzed by positive ionization MS. Without intending to be bound by theory, it appears that at the low pH of the mobile phase of the RP-LC the carboxyl group of the 2-AA label is protonated and, hence, neutral, thereby resulting in a consequential higher hydrophobicity on the reversed phase column, which in turn leads to stronger retention of the 2-AA labeled N-glycans compared to 2-AB labeled glycans.

Also, the use of 2-AA as a label of neutral or acidic glycans, leads to efficient ionization in positive ionization mode of the mass spectrometer when the separated labeled glycans are subsequently analyzed via MS.

Furthermore, high injection volumes are possible when using the method of the invention, which means that comparatively large samples (*e.g.,* 100 µl) can be injected into the HPLC system without further preparative steps after sample preparation, *e.g.,* by gel-filtration. This is advantageous compared to the widely established HILIC method where only a few µl can be injected or the sample has to be concentrated and diluted in acetonitrile to enable higher injection volumes, hence enabling high sensitivity.

In addition, unlike the published approaches, there is no need for an ion pairing reagent to analyze acidic and neutral glycans in one run (*i*.*e*., when MS is directly coupled to, or "on-line" with, the RP-LC). An ion-pairing reagent (*e.g.,* trifluoroacetic acid; TFA) is an amphiphilic molecule able to make hydrophobic as well as electrostatic interactions with the column surface and the polar analytes, respectively. When MS is directly coupled to the RP-LC one would expect a need for such a reagent to improve retention, *e.g*., of sialylated glycans. Surprisingly, it has been found that an ion-pairing reagent is not required in the context of the present invention. The resulting analytes elute in sharper peaks meaning at higher concentration and better sensitivity. This also allows for a reduced LC run time.

The ionization efficiency in positive electrospray ionization (ESI) is good for neutral as well as for acidic N-glycans in combination with the used acidic mobile phase.

The methods of analyzing glycoprotein glycans claimed herein, and the corresponding uses, thus offer high sensitivity and high resolution. The Examples demonstrate the feasibility of 2-AA as a label for the analysis of neutral and acidic N-glycans of antibodies with positive ESI MS. Compared to RP-LC-MS of 2-AB labeled N-glycans more structural isomers can be separated. The inventors have furthermore found that, surprisingly, the methods claimed herein may also be advantageously applied to other glycoproteins, *e.g*., to erythropoietin. Specifically, the methods claimed herein were found to be effective in separating erythropoietin glycans, which belong to the most complex glycans to be found in therapeutic glycoproteins.

Due to the high resolution of the liquid chromatography the high complexity of glycoprotein glycosylation can be investigated in detail. Through the high injection volume even low abundant N-glycans can be detected and quantified. The selectivity of the reversed phase chromatography separates various structural isomers of different types of N-glycans and more importantly separates the N-glycans in seven different groups, oligomannose, hybrid and complex glycans without core fucosylation and hybrid and complex glycans with core fucosylation as well as two sialic acid containing groups again with and without the core fucose residue.

Where information about the N-glycan linkages cannot be deduced by the method of the invention enzymatical digestion with exoglucosidases may be performed to discriminate between several isomers. Hybrid structures with differences in the hexose number can thus be solved.

Apart from the afore-mentioned advantages of 2-AA as glycan label compared to, e.g., 2-AB (including stronger retention on the RP column during RP-LC compared to the use of 2-aminobenzamide (2-AB) and efficient ionization and detection of 2-AA labeled N-glycans during MS detection while allowing decreased LC run time), the inventors have also found that the acidic conditions during RP-LC lead to an efficient separation of acidic 2-AA N-glycans carrying terminal sialylation without the need for an ion-pairing reagent. 2-AA N-glycans are more sensitive in fluorescence detection and better ionization is achieved compared to methods using 2-AB. Moreover, in contrast to HILIC, no buffered mobile phase containing ammonium is used. This is an additional advantage since ammonium can suppress efficient ionization.

The method of the present invention can be used, for example, to characterize the glycosylation of antibodies or other glycoproteins in detail in a single analytical approach. The novel approach described and claimed herein can be used to create a comprehensive glycan map with the ability to detect and identify even minor portions of the N-glycan pool of a given glycoprotein, such as a monoclonal antibody (mAb).

### Summary of the Invention

Accordingly, the present invention relates to a method of analyzing the glycans of a glycoprotein, comprising (i) labeling the glycans with anthranilic acid (2-AA); and (ii) separating the labeled glycans using reversed-phase liquid chromatography (RP-LC), wherein (a) the mobile phase used during RP-LC is acidic; and (b) the reversed-phase liquid chromatography (RP-LC) is performed under conditions under which the carboxyl group of the 2-AA label attached to the glycans is neutral; and (iii) subjecting the labeled glycans to positive ionization mass spectrometry (MS) after the separation using RP-LC; wherein the glycans are neutral or acidic, and wherein the method does not involve the use of an ion-pairing reagent in the mobile phase. The RP-LC can be directly coupled with the MS analysis such that there is no further analytical or preparatory step between the RP-LC and the MS analysis. However, other analytical or preparatory step may well be included between the RP-LC and the MS analysis step.

In a preferred embodiment of the invention, the glycans are N-glycans of the following types (i) oligomannose; (ii) hybrid; or (iii) complex. In a more preferred embodiment, the N-glycans are G1F isomers, particularly the G1 F isomers with 1,3 or 1,6 galactosylation.

In preferred embodiments, the reversed-phase liquid chromatography is (a) reversed-phase high performance liquid chromatography (RP-HPLC); and/or (b) done on a reversed-phase liquid chromatography column.

In the methods of the present invention, it is preferred that the mobile phase used during RP-LC comprises (a) formic acid; (b) formic acid in an amount from 0.1 % to 2.0%; or (c) formic acid in an amount of 1.0%.

It is also preferred that the temperature under which separation is performed is in the range of 40°C to 60°C, or is 50°C.

In preferred embodiments, it is preferred that the flow rate is in a range of 100-500 µl per minute, or is 300 µl per minute.

In the methods of the invention, the mass spectrometry is preferably ion-trap mass spectrometry, particularly positive ionization mass spectrometry.

In preferred embodiments, the mobile phase has a pH (a) in the range of 1 to 4; (b) in the range of 1.5 to 3; (c) in the range of 1.8 to 2.9; (d) in the range of 1.9 to 2.75 (e) in the range of 2 to 2.7; (f) in the range of 2.1 to 2.18; (g) of 2.1; or (h) of 2.18.

In the methods of the invention, the glycoprotein is preferably (a) an antibody; (b) a monoclonal antibody; (c) a monoclonal antibody of an IgG type; or (d) a monoclonal antibody of an IgG type selected from IgG1, IgG2, IgG3, or IgG4; or (e) erythropoietin.

The present invention furthermore relates to the use of 2-AA in any of the methods according to the invention. The RP-LC may be directly coupled with the MS analysis (although in other analytical or preparatory step may well be included).

As noted above, the present invention allows the analysis of anthranilic acid labeled glycans, particularly N-glycans, with high sensitivity and selectivity. The methods and uses according to the invention allow the analysis of acidic and neutral N-glycans in one approach, using a highly MS compatible mobile phase for liquid chromatography. The mass spectrometer can be operated in positive ionization for both neutral and acidic N-glycans.

The use of anthranilic acid according to the invention to label N-glycans with a more acidic mobile phase is advantageous compared to the use of 2-AB in RP-LC. At lower pH values, the carboxylic acid group of 2-AA is protonated, and thus, neutral, thus having a higher hydrophobicity and therefore a stronger retention on the reversed phase column. The on-line mass spectrometry detection with positive ionization is also favored under acidic conditions due to the high proton concentration. This allows efficient ionization and detection of 2-AA labeled N-glycans. The acidic conditions also lead to an efficient separation of acidic 2-AA N-glycans carrying terminal sialylation, without the need of an ion-pairing reagent.

The use of formic acid instead of, e.g., acetic acid, as a component of the mobile phase, further improves the separation. The low pH of the mobile phase achieved with the formic acid leads to an improved retention of the 2-AA labeled N-glycans and to an efficient ionization in positive ionization mode of the mass spectrometer.

The present invention provides high selectivity for N-glycan isomers for all types of N-glycans (oligomannose, hybrid and complex). 2-AA provides better separation in RP-HPLC that allows shorter run times on a RP column. This allows positive electrospray ionization mass spectrometry (ESI-MS) of both neutral and acidic glycans and acidic glycans to elute according to their family. Even sialic acid containing glycans are easily detected with high intensity. For example, effective separation of the highly abundant G1 F isomers of mAbs is achieved.

### Brief Description of the Figures

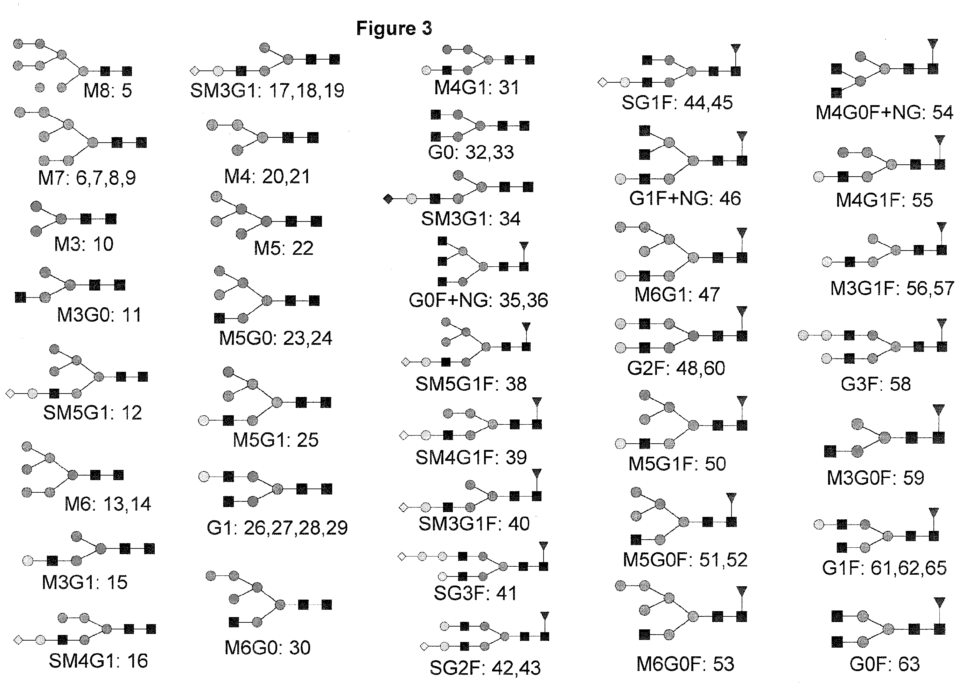

**Figure 1**
   Fluorescence chromatogram of 2-AB N-glycans separated on a RP column. Zoomed view (B) of the chromatogram shows the smaller peaks of less abundant N-glycans. The numbered peaks were identified using MS and MS². Stacked numbering indicates co-elution of N-glycans. Table 1 lists the MS data of identified peaks. The 2-AB glycans elute in different groups according to their type. Oligomannose glycans elute first, followed by acidic hybrid and complex type as well as neutral hybrid glycans. Acidic hybrid with core fucose elute after complex glycans. Neutral hybrid structures co-elute between approx. 57 and 86 min with acidic complex followed by complex 2-AB glycans, all three groups carrying a core fucose.
**Figure 2**
   RP chromatogram of the mAb glycan standard showing the grouping of the eluting 2-AA N-glycans. (A) High Mannose structures elute first, followed by non-fucosylated hybrid and complex glycans. Fucosylated hybrid and complex structures elute last in the chromatogram. Acidic glycans elute right before their appropriate neutral glycans. (B) Zoom of the region between 18 and 42 min showing the less abundant glycans. The identified glycans are numbered and the appropriate masses are listed in Table 1. Stacked numbering indicates co-elution of N-glycans. The glycan structures are depicted in Figure 3.
**Figure 3**
   Symbol structures of the identified N-glycans. The numbering is according to the peak numbering for the N-glycans in the fluorescence chromatograms and in Table 1. For structures with multiple peak assignments only one possible isomer is drawn. Symbols: ▼ L-Fucose, ■ N-Acetyl-D-glucosamine, ● D-Mannose, 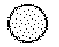 D-Galactose, ◊ N-Glycoyl-neuraminic acid, ◆ N-Acetyl-neuraminic acid.
**Figure 4**
   MS² spectrum of the 2-AA labeled G1 FSG glycan from mAb3 at *m*/*z* 1027.9. The dissociated bonds of the [M+2H]²⁺ ion are depicted and the assigned B and Y ions are labeled in the spectrum.
**Figure 5**
   MS² spectrum of the 2-AA labeled M5G1 FSG glycan from mAb3 at *m*/*z* 1088.4. Resulting single charged B and Y ions from the [M+2H]²⁺ ion are shown. B ions were exclusively from the α-branch containing a GlcNAc (N-acetyl-D-glucosamine) that is able to carry a charge.
**Figure 6**
   MS² spectrum of the 2-AA labeled G3F N-glycan from mAb3. The dissociated bonds of the [M+2H]²⁺ ion are depicted and the assigned B and Y ions are labeled in the spectrum. The glycan accounts for <0.01% of the glycan pool of mAb3.
**Figure 7**
   EICs of four structural M7 isomers from mAb2. Panel A shows the EIC of 2-AB labeled N-glycans. Panel B shows the EIC for the 2-AA labeled glycans. The selectivity is identical for the M7 isomers in both approaches.
**Figure 8**
   EICs of the G1F glycan from mAb2. The 2-AB labeled G1F elutes in one peak (A), whereas the 2-AA labeled glycans (B) are separated into the two isomers. The terminal galactose residue (C) can either be linked to the α1,6 or the α1,3 branch of the bi-antennary N-glycan.
**Figure 9**
   RP FLD chromatogram of the mAb1 N-glycan pool after labeling with 2-AA. Peaks identified by MS and MS² are numbered and listed in Table 1. The appropriate 2-AA glycans are shown in Table 1. A zoomed view (small window) of the chromatogram shows the smaller peaks.
**Figure 10**
   RP FLD chromatogram of the mAb2 N-glycan pool after labeling with 2-AA. Peaks identified by MS and MS² are numbered and listed in Table 1. The appropriate 2-AA glycans are shown in Figure 3. A zoomed view (small window) of the chromatogram shows the smaller peaks. Stacked numbering indicates co-elution of N-glycans.
**Figure 11**
   RP FLD chromatogram of mAb3 N-glycan pool after labeling with 2-AA. Peaks identified by MS and MS² are numbered and listed in Table 1. The appropriate 2-AA glycans are shown in Figure 3. A zoomed view (small window) of the chromatogram shows the smaller peaks. Stacked numbering indicates co-elution of N-glycans.

### Detailed Description of the Invention

### Definitions

As used herein, *2-AA* is 2-amino benzoic acid, also known as anthranilic acid. For the purposes of the present invention 2-AA includes isotopic 2-AA, for example the stable isotopic ¹²[C₆]-2-AA) or ¹³[C₆]-2-AA variants (Prien, J.M., Prater, B.D., Qin, Q. & Cockrill, S.L. Mass spectrometric-based stable isotopic 2-aminobenzoic acid glycan mapping for rapid glycan screening of biotherapeutics. Analytical Chemistry 82, 1498-508 (2010)). Glycans labelled with such isotopic variants of 2-AA may be analyzed using positive-mode matrix-assisted laser desorption ionization time-of-flight mass spectrometry (MALDI-TOF MS) or on-line negative-mode electrospray mass spectrometry (ESI-MS/MSⁿ) and/or nanospray mass spectrometry (NSI-MS) (Prien, J.M., Prater, B.D., Qin, Q. & Cockrill, S.L. Mass spectrometric-based stable isotopic 2-aminobenzoic acid glycan mapping for rapid glycan screening of biotherapeutics. Analytical Chemistry 82, 1498-508 (2010)).

Carbohydrate moieties are described herein with reference to commonly used nomenclature for oligosaccharides. A review of carbohydrate chemistry which uses this nomenclature can be found, for example, in Hubbard and Ivatt, Ann. Rev. Biochem. 50.555-583 (1981).

For the purposes of the present invention, a *glycan* refers to any sugar or assembly of sugars, in free form or attached to another molecule, (*i.e.*, saccharide or carbohydrate). As referred to herein, an *N-glycan* is a glycan covalently linked to an asparagine residue of a polypeptide chain in the consensus sequence: -Asn-X-Ser/Thr (*e.g*., Manβ1-4GlcNAcβ1-4GlcNAcβ1-N-Asn). As referred to herein, an *acidic glycan* is an N-glycan containing at least one terminal sialic acid (at the non-reducing terminus). As referred to herein, a *neutral glycan* is an N-glycan that does not contain any sialic acid. As referred to herein, *glycosylation* is the enzyme-catalyzed covalent attachment of a carbohydrate to a polypeptide, lipid, polynucleotide, carbohydrate, or other organic compound, generally catalyzed by glycosyltransferases, utilizing specific sugar nucleotide donor substrates. For the purposes of the present invention, a *polysaccharide* is a glycan composed of repeating monosaccharides, preferably greater than ten monosaccharide units in length. As referred to herein, a *sugar* refers to any carbohydrate, preferably to low molecular weight carbohydrates that are sweet in taste (see glossary of Essentials of Glycobiology. 2nd edition. Varki A, Cummings RD, Esko JD, et al., editors. Cold Spring Harbor (NY): Cold Spring Harbor Laboratory Press; 2009).

For the purposes of the present invention, the term *glycoprotein* refers to peptides and proteins, including antibodies, having at least one glycan side chain. Preferred glycoproteins are glycan containing therapeutic proteins that have been expressed in higher eukaryotic cells. In this regard, exemplary useful higher eukaryotic cells are selected from the following cell lines:

| Cell Line | Meaning | Origin | Tissue Origin | Morphology |
|---|---|---|---|---|
| CHO | Chinese hamster ovary | Hamster | Ovary | Epithelium |
| COS-7 | *Cercopithecus aethiops,* origin-defective SV-40 | Ape - *Cercopithecus aethiops (Chlorocebus)* | Kidney | Fibroblast |
| BHK-21 | Baby hamster kidney fibroblast cells | Hamster | Kidney | Fibroblast |
| HEK-293 | Human embryonic kidney | Human | Kidney (embryonic) | Epithelium |
| HeLa | "Henrietta Lacks" | Human | Cervical cancer | Epithelium |
| HL-60 | Human leukemia | Human | Myeloblast | Blood cells |
| HUVEC | Human umbilical vein endothelial cell | Human | Umbilical vein endothelium | Epithelial |
| Jurkat | | Human | T cell leukemia | white blood cells |
| MCF-7 | Michigan Cancer Foundation-7 | Human | Mammary gland | Invasive breast ductal carcinoma |
| NIH-3T3 | NIH, 3-day transfer, inoculum 3 x 10⁵ cells | Mouse | Embryo | Fibroblast |
| RenCa | Renal carcinoma | Mouse | | Renal carcinoma |
| U937 | | Human | Leukaemic monocytic lymphoma | |
| Vero cells | *Vero* (truth) | African green monkey | Kidney epithelium | |

Particularly preferred in this regard are CHO cells, which are widely used in the art to express biopharmaceutically useful glycoproteins, such as antibodies.

The glycoproteins may be homologous to the host cell, or may be heterologous, *i.e.,* foreign, to the host cell being utilized, such as, for example, a human glycoprotein produced by a Chinese hamster ovary (CHO) host cell. Such glycoproteins are generally referred to as *recombinant glycoproteins.*

In certain embodiments, the glycoproteins expressed by a host-cell are directly secreted into the medium.

Examples of suitable mammalian, and in particular human, glycoproteins include the following molecules:
a cytokine; a cytokine receptor; a chemokine, such as TNF and TECK; a chemokine receptor, such as a TNFR and CCR9; a growth hormone, such as human growth hormone and bovine growth hormone; growth hormone releasing factor; parathyroid hormone; thyroid stimulating hormone; a lipoprotein; alpha-1-antitrypsin; insulin A-chain; insulin B-chain; proinsulin; follicle stimulating hormone; calcitonin; luteinizing hormone; glucagon; human macrophage inflammatory protein (MIP-1-alpha); a serum albumin, such as human serum albumin; mullerian-inhibiting substance; relaxin A-chain; relaxin B-chain; prorelaxin; mouse gonadotropin-associated peptide; a clotting factor, such as factor VIIIC, factor IX, tissue factor, and von Willebrand factor; an anti-clotting factor, such as Protein C; atrial natriuretic factor; lung surfactant; a plasminogen activator, such as urokinase or human urine or tissue-type plasminogen activator (t-PA); bombesin; thrombin; hemopoietic growth factor; enkephalinase; RANTES; a microbial protein, such as beta-lactamase; DNase; inhibin; activin; vascular endothelial growth factor (VEGF); a receptor for hormones or growth factors; an integrin; protein A or D; a rheumatoid factor; a neurotrophic factor such as bone-derived neurotrophic factor (BDNF), neurotrophin-3, -4, -5, or -6 (NT-3, NT-4, NT-5, or NT-6); a nerve growth factor, such as NGF-beta; platelet-derived growth factor (PDGF); a fibroblast growth factor, such as aFGF and bFGF; epidermal growth factor (EGF); a transforming growth factor (TGF) such as TGF-alpha and TGF-beta, including TGF-beta1, TGF-beta2, TGF-beta3, TGF-beta4, or TGF-beta5; insulin-like growth factor-I and -II (IGF-I and IGF-II); des(I-3)-IGF-I (brain IGF-I); an insulin-like growth factor binding protein, a CD protein, such as CD-3, CD-4, CD-8, and CD-19; erythropoietin; an osteoinductive factor, an immunotoxin; a bone morphogenetic protein (BMP); an interferon, such as interferon-alpha, -beta, and -gamma; a colony stimulating factor (CSF), such as M-CSF, GM-CSF, and G-CSF; an interleukin (ILs), such as IL-1 to IL-21; superoxide dismutase; a T-cell receptor; a cell surface membrane protein; a transport protein; a homing receptor; a regulatory protein; a decay accelerating factor; and a viral antigen, such as, a portion of the HIV-1 or HIV-2 envelope protein.

A preferred glycoprotein in the context of the present invention is a hemopoietic growth factor, such as erythropoietin, or a colony stimulating factor (CSFs), such as M-CSF, GM-CSF, and G-CSF. Particularly preferred in this regard is erythropoietin.

Another preferred glycoprotein in the context of the present invention is an antibody.

The term *antibody* as referred to herein includes whole antibodies and any antigen binding fragment (*i.e.*, *antigen-binding portion*) or single chain thereof. An *antibody* refers to a glycoprotein comprising at least two heavy (H) chains and two light (L) chains inter-connected by disulfide bonds, or an antigen binding portion thereof. Each heavy chain is comprised of a heavy chain variable region (abbreviated herein as V_{H}) and a heavy chain constant region. The heavy chain constant region is comprised of three domains, CH1, CH2 and CH3. Each light chain is comprised of a light chain variable region (abbreviated herein as \/_{L}) and a light chain constant region. The light chain constant region is comprised of one domain, C_{L}. The V_{H} and V_{L} regions can be further subdivided into regions of hypervariability, termed complementarity determining regions (CDR), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (*e.g.,* effector cells) and the first component (C1q) of the classical complement system.

Examples of antigen-binding fragments encompassed within the term *antigen-binding portion* of an antibody include (i) a Fab fragment, a monovalent fragment consisting of the V_{L}, V_{H}, CL and CH1 domains; (ii) a F(ab')2 fragment, a bivalent fragment comprising two Fab fragments linked by a disulfide bridge at the hinge region; (iii) an Fd fragment consisting of the V_{H} and CH1 domains; (iv) an Fv fragment consisting of the V_{L} and V_{H} domains of a single arm of an antibody, (v) a dAb fragment (Ward et al., Nature. 341:544-546 (1989)), which consists of a V_{H} domain; and (vi) an isolated complementarity determining region (CDR) or (vii) a combination of two or more isolated CDRs which may optionally be joined by a synthetic linker. Furthermore, although the two domains of the Fv fragment, V_{L} and V_{H}, are coded for by separate genes, they can be joined, using recombinant methods, by a synthetic linker that enables them to be made as a single protein chain in which the V_{L} and V_{H} regions pair to form monovalent molecules (known as single chain Fv (scFv); see *e.g.,* Bird et al. Science. 242:423-426 (1988); and Huston et al. Proc. Natl. Acad. Sci. USA 85:5879-5883 (1988). Such single chain antibodies are also intended to be encompassed within the terms *antigen-binding portion* and *antigen-binding fragment* of an antibody. These antibody fragments are obtained using conventional techniques known to those with skill in the art, and the fragments are screened for utility in the same manner as are intact antibodies.

The term *monoclonal antibody* (mAb), as used herein, refers to an antibody which displays a single binding specificity and affinity for a particular epitope. Accordingly, the term *human monoclonal antibody* refers to an antibody which displays a single binding specificity and which has variable and constant regions derived from human germline immunoglobulin sequences. In one embodiment, human monoclonal antibodies are produced by a hybridoma which includes a B cell obtained from a transgenic non-human animal, *e.g*., a transgenic mouse, having a genome comprising a human heavy chain transgene and a light chain transgene fused to an immortalized cell.

The term *recombinant human antibody,* as used herein, includes all human antibodies that are prepared, expressed, created or isolated by recombinant means, such as (a) antibodies isolated from an animal (*e.g*., a mouse) that is transgenic or transchromosomal for human immunoglobulin genes or a hybridoma prepared therefrom, (b) antibodies isolated from a host cell transformed to express the antibody, *e.g*., from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies have variable and constant regions derived from human germline immunoglobulin sequences. In certain embodiments, however, such recombinant human antibodies can be subjected to *in vitro* mutagenesis (or, when an animal transgenic for human Ig sequences is used, *in vivo* somatic mutagenesis) and thus the amino acid sequences of the V_{H} and V_{L} regions of the recombinant antibodies are sequences that, while derived from and related to human germline V_{H} and V_{L} sequences, may not naturally exist within the human antibody germline repertoire *in vivo.*

As used herein, a *heterologous antibody* is defined in relation to the transgenic non-human organism producing such an antibody. This term refers to an antibody having an amino acid sequence or an encoding nucleic acid sequence corresponding to that found in an organism not consisting of the transgenic non-human animal, and generally from a species other than that of the transgenic non-human animal.

As used herein, *specific binding, selective binding* and *selectively binds,* refer to an antibody or a fragment thereof, binding to a predetermined antigen. For example, in one embodiment, the antibody binds with an affinity (K_{D}) of approximately less than 10⁻⁷ M, such as approximately less than 10⁻⁸ M, 10⁻⁹ M or 10⁻¹⁰ M or even lower when determined by surface plasmon resonance (SPR) technology in a BIACORE 3000 instrument using an analyte and the corresponding antibody as the ligand, and binds to the predetermined antigen with an affinity that is at least two-fold greater than its affinity for binding to a non-specific antigen (*e.g*., BSA, casein) other than the predetermined antigen or a closely-related antigen. The phrases *an antibody recognizing an antigen* and *an antibody specific for an antigen* are used interchangeably herein with the term *an antibody which selectively binds to an antigen.*

As used herein, *reversed phase LC* or *HPLC (RP-LC* or *RP-HPLC)* has a non-polar stationary phase and an aqueous, moderately polar mobile phase, e.g., a silica which has been surface-modified with RMe₂SiCl, where R is a straight chain alkyl group such as C₁₈H₃₇ or C₈H₁₇ (Lehto J and Hou X. Chemistry and Analysis of Radionuclides. Wiley-VCH Verlag & Co., Weinheim, Germany, 2011, page 170).

For the purposes of the present invention, a *mobile phase* of RP-LC or RP-HPLC is preferably a gradient of an organic modifier (*e.g*., acetonitrile or methanol) in water, with an ionic modifier that controls the pH and ionization state or acts as an *ion pairing reagent.* Anionic ion-pair reagents (*e.g*., trifluoroacetic acid (TFA)) bind to protonated basic groups of peptides. The addition of 0.1% TFA acidifies the eluent which causes the carboxylic groups of peptides and proteins to become protonated, resulting in a larger hydrophobicity of the molecules. Cationic ion-pairing reagents (*e.g.,* triethylammonium ions) bind to ionized carboxyl groups of peptides ("Protein Liquid Chromatography". Journal of Chromatography Library, vol. 61. Edited by Kastner M, Elsevier Science B.V., 2000, page 153). Diethylamine (DEA) can also be used as an ion pairing reagent (Melmer et al., Journal of Chromatography A (2011), Volume: 1218(1): 118-123).

As used herein, *ion trap mass spectrometry* is an arrangement in which ions with a desired range of quotients mass/charge are first made to describe stable paths under the effect of a high-frequency electric quadrupole field, and are then separated and presented to a detector by adjusting the field so as to selectively induce path instability according to their respective mass/charge ratios e.g., quadrupole ion trap (see http://www.genomicglossaries.com/content/mass_spectrometry.asp).

For the purposes of the present invention, all *N-glycans* are understood to have the common core sugar sequence, Manal-6(Manα1-3)Manβ1-4GlcNAcβ1-4GlcNAcβ1-Asn-X-Ser/Thr, and are classified into three types: (1) *oligomannose,* in which only mannose residues are attached to the core; (2) *complex,* in which "antennae" initiated by N-acetylglucosaminyltransferases (GlcNAcTs) are attached to the core; and *(3) hybrid,* in which only mannose residues are attached to the Manα1-6 arm of the core and one or two antennae are on the Manα1-3 arm (Essentials of Glycobiology 2nd edition Varki A, Cummings RD, Esko JD, et al., editors. Cold Spring Harbor (NY): Cold Spring Harbor Laboratory Press; 2009; Chapter 8).

For the purposes of the present invention, a reference to the analysis of acidic or neutral glycans, N-glycans, or glycans in general, *in one run, in one approach,* or *in a single (analytical) approach* means that the MS analysis is directly coupled to (or in other words, is performed on-line with) the RP-LC step. This means that there is no further analytical or preparatory step between the RP-LC and the MS analysis. It will be understood that this does not exclude that fluorescence detection will occur between the RP-LC and the MS. Indeed, fluorescence detection of the 2-AA labeled glycans separated on the RP-column will normally (and advantageously) occur before they leave the LC system and proceed to MS, since the fluorescence detector (FLD) that is passed by the labeled glycans is typically a module of the HPLC/LC system.

For the purposes of the present invention, a reference to % will be understood to refer to (v/v) unless explicitly stated otherwise.

As used herein, the term *about* when used together with a numerical value (e.g., a pH value or a percentage value) is intended to encompass a deviation of 20%, preferably 10%, more preferably 5%, even more preferably of 2%, and most preferably of 1% from that value. When used together with a numerical value it is at the same time to be understood as individually disclosing that exact numerical value as a preferred embodiment in accordance with the present invention.

As used herein, the term *comprising* is to be construed as encompassing both "including" and "consisting of", both meanings being specifically intended, and hence individually disclosed, embodiments in accordance with the present invention.

It will be appreciated that the labelling with 2-AA in accordance with the methods and uses claimed herein is particularly advantageous for mapping (*i.e.*, analyzing) neutral or acidic glycans of a glycoprotein, such as an antibody or erythropoietin. The glycans to be analyzed are preferably N-glycans, particularly N-glycans that are either of the oligomannose, hybrid or complex type. As noted earlier herein, the methods and uses of the present invention *inter alia* have the advantage that they are capable of efficiently distinguishing between the G1F isomers of N-glycans, particularly the G1F isomers with 1,3 or 1,6 galactosylation.

The labeling in accordance with the methods and uses described and/or claimed herein may be done before the glycans have been removed from the glycoprotein. Preferably, however, the labeling is done after the glycans have been removed from the glycoprotein.

Removal of the glycans may be done chemically or enzymatically by methods well known to those skilled in the art.

For example, chemical removal of glycans for subsequent labeling (or analysis, as the case may be) may be effected by hydrazinolysis or by alkaline β-elimination, methods that are well known to those of skill in the art. An exemplary useful kit for chemical removal is the GlycoProfile™ IV Chemical Deglycosylation Kit offered by Sigma-Aldrich.

Removal of the glycans by enzymatic methods causes no problems to those of skill in the art. A method of glycan removal for subsequent labeling (or analysis) is digestion with PNGaseF (Peptide N-glycosidase F). Various suitable PNGaseF enzymes are offered commercially under different trade names (*e.g.,* N-Glycanase®), which are mostly engineered or optimized PNGaseF, although using an engineered or optimized PNGaseF is not mandatory in the context of the present invention. Enzymatic removal may also be done by using Endoglycosidase H (or an enzyme with similar enzymatic activity, such as IgGZERO™) or Endoglycosidase F2, which cleave between the two N-Acetylglucosamines of the glycan core leaving the first monosaccharide attached to the protein. However, the information whether the glycan carried a fucose or not is lost in this way. Furthermore Endoglycosidase H and Endoglycosidase F2 are only specific for oligomannose and hybrid bi-antennary glycans.

The reversed-phase liquid chromatography to be performed in connection with the methods and uses described and/or claimed herein is preferably reversed-phase high performance liquid chromatography (RP-HPLC), or ultra performance liquid chromatography (UPLC). In this case, as in other preferred cases of RP-LC performed in connection with the present invention, these chromatography methods are performed on a reversed-phase liquid chromatography column.

In a preferred embodiment of the methods and uses described and/or claimed herein, the mobile phase used during RP-LC comprises formic acid. In this regard, preferred amounts of formic acid in the mobile phases are from about 0.1% to about 2.0% formic acid. Typical preferred amounts therefore include about 0.2%, about 0.3%, about 0.4%, about 0.5%, about 0.6%, about 0.7%, about 0.8%, about 0.9%, about 1.0%, about 1.1%, about 1.2%, about 1.3%, about 1.4%, about 1.5%, about 1.6%, about 1.7%, about 1.8%, or about 1.9% formic acid.

An amount of about 1.0% formic acid in the mobile phase is particularly preferred. Herein described are methods and uses, wherein, the mobile phase used during RP-LC comprises acetic acid. In this regard, amounts of acetic acid in the mobile phases are again from about 0.1 % to about 2.0% acetic acid. Typical amounts therefore include about 0.2%, about 0.3%, about 0.4%, about 0.5%, about 0.6%, about 0.7%, about 0.8%, about 0.9%, about 1.0%, about 1.1%, about 1.2%, about 1.3%, about 1.4%, about 1.5%, about 1.6%, about 1.7%, about 1.8%, or about 1.9% acetic acid.

Further, suitable pH values of the mobile phase used during RP-LC are in the range of about 1 to about 4, more preferably in the range of about 1.5 to about 3, yet more preferably of about 1.8 to about 2.9, even more preferably of about 1.9 to about 2.75, and particularly preferably of about 2 to about 2.7. Preferred is in particular a pH value in the range of about 2.1 to about 2.18. Accordingly, preferred mobile phases used during RP-LC in accordance with the methods and uses described and/or claimed herein will have a pH value of about 1.9, 2, 2.1, 2.2, 2.3, 2.4, 2.5, 2.6, or 2.7, with pH values of about 2.1 or of about 2.18 being particularly preferred.

In the course of the methods and uses claimed herein the separation of the 2-AA labeled glycans by RP-LC may be advantageously performed at temperatures in the range of about 4°C to about room temperature, or at room temperature (with room temperature being defined as 23°C for the purpose of the present invention). Preferably, however, the separation is performed at temperatures above room temperature. Preferred in this regard is a temperature in the range of about 40°C to about 60°C, with about 50°C being particularly preferred.

Suitable flow rates for an RP-LC column in accordance with the present invention will be readily known or determined by those of skill in the art. Generally, suitable flow rates will typically be in a range of about 50-1000 µl per minute. Preferred are, for example, flow rates in a range of about 100-500 µl per minute. Accordingly, preferred values for the flow rate include about 100 µl per minute, about 200 µl per minute, about 300 µl per minute, about 400 µl per minute, or about 500 µl per minute, with a flow rate of about 300 µl per minute being particularly preferred.

As noted above, the methods and uses claimed herein comprise subjecting the 2-AA labeled glycans to positive ionization mass spectrometry (MS) after their separation via RP-LC by directly coupling RP-LC with the MS analysis.

It will be appreciated that the antibody, the glycans of which are analyzed by virtue of the methods and uses described and/or claimed herein, may be any type of antibody, as defined above. Particularly preferred are monoclonal antibodies, such as monoclonal antibodies of the IgG type. Thus, a suitable antibody for analysis in accordance with the present invention may be an IgG antibody selected from the group consisting of IgG1, IgG2, IgG3, and IgG4.

The invention is further illustrated by the following Figures and Examples, which are not to be considered as being limiting for the scope of protection conferred by the claims of the present application.

### Examples

### Example 1: Materials and Methods

### Materials

PNGase F was from New England Biolabs. Acetonitrile (ACN) and acetic acid was from Merck. Formic acid and Sodiumcyanoborohydride was from Fluka. PD MiniTrap™ Sephadex® G-10 columns were from GE Healthcare. DMSO was from Sigma. Amicon Ultra 30K filter devices were from Milipore. The mAb glycan standard was prepared at Sandoz. Monoclonal antibodies 1-3 were obtained from in-house development at Sandoz.

### Methods

### Enzymatic N-glycan release using PNGaseF

1 mg of desalted mAb was used. The N-glycans (15 nmol) were released using PNGaseF by incubating the samples overnight (-17 hours) at 37°C. N-glycans were separated from the proteins using Amicon 30K filter devices and were brought to dryness using a speedvac.

### Fluorescence labeling of released N-glycans

Na[BH3(CN)] and either 2-AA or 2-AB were dissolved in 70% DMSO : 30% acetic acid to obtain concentrations of 63 mg/ml and 50 mg/ml, respectively. 15 µl of the labeling solution and 10 µl of deionized water were added to approximately 15 nmol dryed glycans. Labeling reaction was performed at 37°C for 17 h.

Excess label was removed by gel filtration using G-10 columns. Conditioning of the columns was done with 10 ml H₂O. Samples were filled up to 100 µl with deionized water and the sample was applied to the column. After rinsing the column with 700 µl H₂O the purified fluorescence labeled N-glycans were eluted with 600 µl H₂O.

### Reversed Phase HPLC of labeled N-Glycans

Liquid chromatography was performed using an Agilent 1200 Series chromatograph with a Waters ACQUITY UPLC BEH130 C18 (2.1x150 mm 1.7 µm) column. For 2-AA glycan analytics mobile phase A consisted of 1.0% formic acid in H₂O, mobile phase B consisted of 50% ACN in 1.0% formic acid in H₂O. Oven temperature was 50°C and the flow-rate was 0.30 ml/min. The column was equilibrated with 4% B. After injection of up to 100 µl sample the mobile phase composition was held at 4% B for two minutes. B was then raised in four steps to 28%. First to 10% over 27 minutes, then to 11.5% over 10 minutes, then to 14% over 8 minutes and finally to 28% over 19 minutes. The column was regenerated by raising B to 90% over 4 minutes, followed by an isocratic gradient over 2 minutes. The column was then reequilibrated at 8% B for 5 minutes. Fluorescence detection was performed with an excitation wavelength of 250 nm and an emission wavelength of 425 nm. For analysis of 2-AB labeled glycans the mobile phase consisted of 0.5% formic acid in H₂0 (mobile Phase A) and 0.5% formic acid and 5% ACN in H₂0 (mobile Phase B). Oven temperature was 40°C and the flow rate was 0.3 ml/min. The column was equilibrated with 25% B. After injection the mobile phase was held at 25% B for 2 minutes, B was increased to 55% over 60 minutes and then increased to 61% over 24 minutes. The composition was held for 2 minutes and then initial condition was reached after 2 minutes and held for additional 5 minutes. Fluorescence detection was performed with an excitation wavelength of 250 nm and an emission wavelength of 428 nm.

### Mass Spectrometry

The HPLC was directly coupled to a 3D ion trap ESI-MS (Bruker AmaZon). The ion trap was operated in Enhanced Resolution Mode with a capillary voltage of 4 kV. The capillary temperature was set to 250°C with a nebulizer pressure of 2 bar and a dry gas flow of 6 l/min. MS² spectra were generated with the Auto MS² mode using Collision Induced Dissociation (CID).

### Example 2: Results and Conclusions

### Results

### LC-MS of 2-AB labeld N-glycans

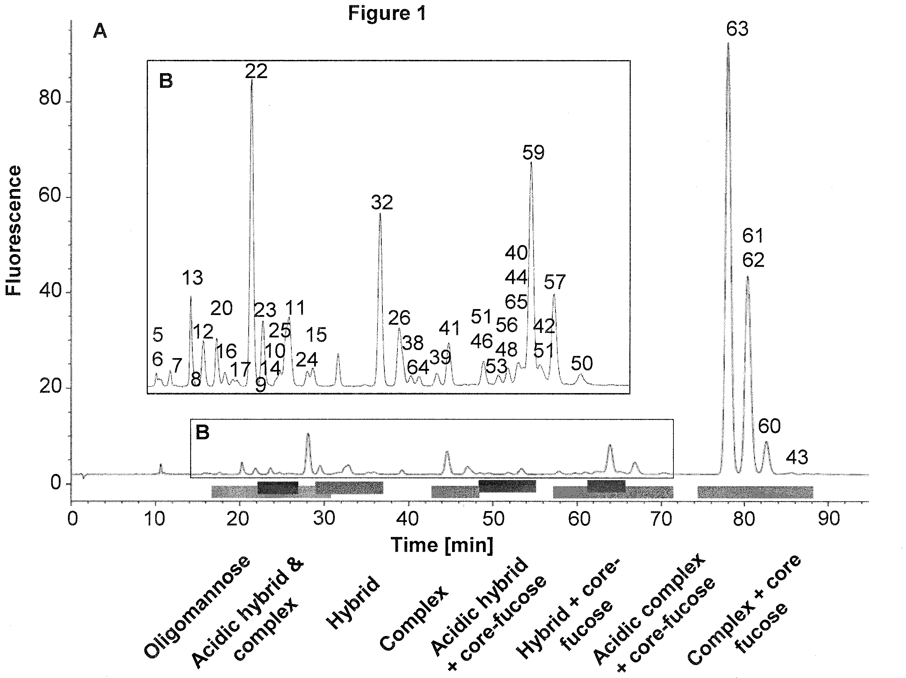

The RP LC-MS platform used encompassed two structurally closely similar chemical labels, 2-AB and 2-AA. In a first step a RP LC-MS method for 2-AB labeled N-Glycans was developed. 2-AB labeled N-glycans reveal weak retention properties on C18 columns, therefore a mobile phase gradient with low content of organic solvent was used. The separation was optimal with a mobile phase composition of 0.5% formic acid in water for eluent A and 5% acetonitrile in 0.5% formic in water for eluent B. The resulting fluorescence chromatogram of the 2-AB RP-LC-MS method for the mAb glycan standard is shown in Figure 1. The glycans elute in groups. In order to shorten the run time the mobile phase composition was adapted. The use of formic acid instead of acetic acid improved the retention and led to sharper peaks. A run time of 95 minutes was sufficient to analyze the N-glycans of a mAb.

The first compounds eluting between 16-30 minutes from the column were the high mannose glycans, starting with high to low number of mannose residues (Figure 1). The acidic hybrid and complex glycans (Figure 1) overlapped with the oligomannose group from approximately 22-26 minutes. The next glycans that eluted belonged to the hybrid N-glycans lacking the core-fucose at the terminal GlcNAc (Figure 1), eluting from 28-36 minutes. The complex bi-antennary 2-AB glycans eluted in the middle of the chromatogram (Figure 1, 42-48 min.) right before the acidic hybrid glycans with core-fucose. Hybrid (Figure 1, 57-71 min.) and acidic complex (Figure 1, 48-66 min.) 2-AB glycans, both groups with a fucose residue attached to their core, co-eluted. The group with the most abundant glycans, the complex type glycans with core-fucose, eluted at the end of the chromatogram with a retention time of 74-88 minutes. Sialic acid containing 2-AB labeled glycans eluted in sharp peaks before their corresponding neutral glycans.

Labeled oligomannose and hybrid structures eluted from high to low number of monosaccharides whereas complex type 2-AB glycans, including the acidic variants, eluted from low to high number of monosaccharide units. Several 2-AB glycans eluted with similar retention time and could not be separated. The 2-AB labeled glycans were identified by MS and MS² using the ion-trap mass spectrometer. Table 1 depicted below contains the MS data for the identified glycans and the respective glycan structures are drawn in Figure 3.

**Table 1: 2-AA and 2-AB labeled glycans from mAb glycan standard identified by MS and MS² in the ion-trap. Oberserved and theoretical mass are shown for each assigned glycan. For N-glycan isomers only one mass is shown examplarily. The peak numbers correspond to the appropriate peak numbering in the chromatograms and to the structures in Figure 3.**

| | 2-AA Glycans | | 2-AB Glycans | | |
|---|---|---|---|---|---|
| Peak | Observed Mass | Theoretical Mass | Observed Mass | Theoretical Mass | N-Glycan |
| 5 | 1841.602 | 1841.645 | 1840.689 | 1840.661 | M8 |
| 6,7,8,9 | 1679.541 | 1679.592 | 1678.604 | 1678.608 | M7 |
| 10 | 1031.406 | 1031.381 | 1030.425 | 1030.397 | M3 |
| 11 | 1234.470 | 1234.460 | 1233.489 | 1233.476 | M3G0 |
| 12 | 2027.716 | 2027.709 | 2026.739 | 2026.725 | SM5G1 |
| 13,14 | 1517.592 | 1517.539 | 1516.573 | 1516.555 | M6 |
| 15 | 1396.550 | 1396.513 | 1395.54 | 1395.529 | M3G1 |
| 16 | 1855.655 | 1865.656 | 1865.678 | 1864.672 | SM4G1 |
| 17,18,19 | 1703.619 | 1703.603 | 1702.615 | 1702.619 | SM3G1 |
| 20,21 | 1193.457 | 1193.433 | 1192.49 | 1192.449 | M4 |
| 22 | 1355.525 | 1355.486 | 1354.533 | 1354.502 | M5 |
| 23,24 | 1558.617 | 1558.566 | 1557.582 | 1557.582 | M5G0 |
| 25 | 1720.647 | 1720.618 | 1719.643 | 1719.634 | M5G1 |
| 26,27,28,29 | 1599.618 | 1599.592 | 1598.612 | 1598.608 | G1 |
| 30 | 1720.647 | 1720.618 | 1719.643 | 1719.634 | M6G0 |
| 31 | 1558.592 | 1558.566 | 1557.576 | 1557.582 | M4G1 |
| 32,33 | 1437.586 | 1437.539 | 1436.565 | 1436.555 | G0 |
| 34 | 1687.579 | 1687.608 | - | 1686.624 | SM3G1 |
| 35,36 | 1786.675 | 1786.677 | 1785.708 | 1785.693 | G0F+GN |
| 37 | 1583.710 | 1583.597 | 1582.648 | 1582.613 | G0F |
| 38 | 2173.726 | 2173.767 | 2172.78 | 2172.783 | SM5G1F |
| 39 | 2011.693 | 2011.714 | 2010.754 | 2010.73 | SM4G1F |
| 40 | 2376.763 | 2376.846 | 2375.787 | 2375.862 | SG3F |
| 41 | 1849.705 | 1849.661 | 1848.692 | 1848.677 | SM3G1F |
| 42,43 | 2214.754 | 2214.793 | 2213.818 | 2213.809 | SG2F |
| 44,45 | 2052.732 | 2052.740 | 2051.768 | 2051.756 | SG1F |
| 46 | 1948.741 | 1948.729 | 1947.804 | 1947.745 | G1F+NG |
| 47 | 2028.702 | 2028.729 | - | 2027.745 | M6G1F |
| 48,49 | 1907.689 | 1907.703 | 1906.775 | 1906.719 | G2F |
| 50 | 1866.679 | 1866.676 | 1865.698 | 1865.692 | M5G1F |
| 51,52 | 1704.609 | 1704.624 | 1703.644 | 1703.64 | M5G0F |
| 53 | 1866.650 | 1866.676 | 1865.668 | 1865.692 | M6G0F |
| 54 | 1583.533 | 1583.597 | 1582.608 | 1582.613 | M3G0F+NG |
| 55 | 1704.609 | 1704.624 | 1703.643 | 1703.64 | M4G1F |
| 56,57 | 1542.566 | 1542.571 | 1541.582 | 1541.587 | M3G1F |
| 58 | 2069.748 | 2069.756 | - | 2068.772 | G3F |
| 59 | 1380.553 | 1380.518 | 1379.569 | 1379.534 | M3G0F |
| 60 | 1907.690 | 1907.703 | 1906.746 | 1906.719 | G2F |
| 61,62 | 1745.610 | 1745.650 | 1745.655 | 1744.666 | G1F |
| 63 | 1583.709 | 1583.597 | 1582.607 | 1582.613 | G0F |

### Separation of 2-AA labeled N-glycans

As alternative to the 2-AB labeled glycans also 2-AA labeled glycans were tested with the developed 2-AB method. Most 2-AA glycans remained on the column after the 95 minute gradient, only the oligomannose structures eluted late. Due to this stronger retention of the 2-AA labeled glycans the mobile phase had to be adapted to enable optimal separation. In contrast to 2-AB, 2-AA is negatively charged at neutral pH value. Therefore the composition of the mobile phase was adapted to a lower pH to provide sufficient protons for an efficient ionization in the positive MS mode. At a concentration of 1% formic acid and an ACN content of eluent B increased by a factor of 10 compared to the 2-AB method to 50% optimal separation and good ionization were achieved. The resulting run-time was 78 minutes.

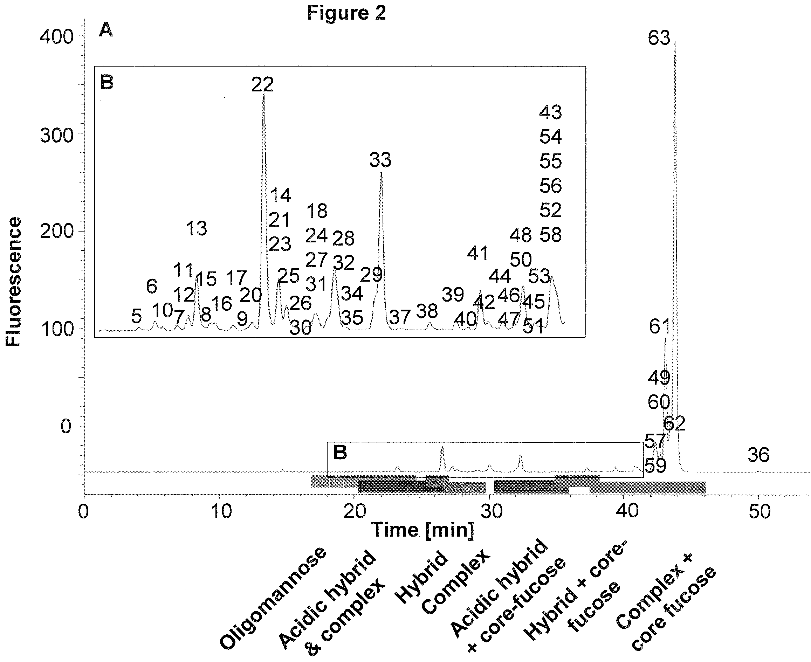

Figure 2 shows the fluorescence chromatogram of the 2-AA labeled mAb glycan standard which is similar to one of the 2-AB method (Figure 1). The labeled glycans elute in different groups, highlighted with different shades of grey in Figure 2. High Mannose structures are the first group eluting from high number to small number of monosaccharide residues, for example from M9 to M5 (19-27 min.). Sialic acid containing non-fucosylated hybrid and complex variants (23-30 min.) are then followed by the neutral non-fucosylated hybrid variants (28-31 min.). Complex structures lacking the core fucose (30-34 min.) elute before sialic acid containing hybrid glycoforms and sialic acid containing complex forms (34-40 min.), both with core fucosylation. Bi-secting structures co-elute with latter. Neutral core-fucosylated hybrid glycans (39-42 min.) elute before the most abundant core fucosylated complex variants in mAbs, the bi-antennary N-glycans (42-51 min.). This grouping is similar to our 2-AB approach and to previous investigations. Sialic acid containing non-fucosylated hybrid and complex variants elute after the high mannose and before the neutral non-fucosylated hybrid group. The same order is observed for the core fucosylated hybrid and complex glycoforms. Again eluting sialic acid containing 2-AA glycans gave the same sharp peak shape as neutral glycans without the need for an ion-pairing reagent. Compared to the 2-AB method, the peaks were sharper and more condensed, but with more overlapping peaks.

### Identification of N-glycans using positive Mode ESI-MS and MS²

The use of 2-AA was so far mostly described for negative ionization MS (D.J. Harvey, Collision-induced fragmentation of negative ions from N-linked glycans derivatized with 2-aminobenzoic acid, Journal of Mass Spectrometry: JMS 40, 642-53 (2005); and J.M. Prien, B.D. Prater, Q. Qin, S.L. Cockrill, Mass spectrometric-based stable isotopic 2-aminobenzoic acid glycan mapping for rapid glycan screening of biotherapeutics, Analytical Chemistry 82, 1498-508 (2010)) due to the negative charge of the acid group. The acidic mobile phase used in this investigation provided good ionization efficiency. 2-AA N-glycans were identified according to their mass derived from MS spectra and by the mass of their appropriate fragments generated by CID in. Table 1 above lists all identified 2-AB and 2-AA N-glycans from the mAb glycan standard with their observed and calculated mass. Some glycan motifs occured several times in course of the chromatogram. These can be structural isomers or bisecting or tri-antennary variants with the same mass that cannot be distinguished as no linkage information is obtained by this LC-MS approach.

The charge states of the labeled glycans increases with increasing mass. The glycans with a mass < 1200 Da occur almost exclusively as [M+H]¹⁺ ions, whereas glycans with > 1200 Da are double charged [M+2H]²⁺, but also single charged [M+H]¹⁺ ions are present for glycans < 1500 Da. At mass ranges > 1800 Da, the N-glycans begin to ionize as triple charged [M+3H]³⁺ ions with the exception of the oligomannose type glycans which exist at most as double charged ions. Adduct ions are present for all charge states and the oligomannose glycans tend to have the highest affinity for adduct formation. Double and triple charged ions occur as mixed adducts too. [M+Na]¹⁺, [M+K]¹⁺, [M+H+Na]²⁺, [M+2Na]²⁺ and [M+H+K]²⁺ are the most abundant adduct ions. On-line MS detection provides more information than only the identification of N-glycans by their mass and fragments, as co-eluting 2-AA N-glycans can be identified and quantified using the extracted ion chromatograms (EICs) of the appropriate *m*/*z* values.

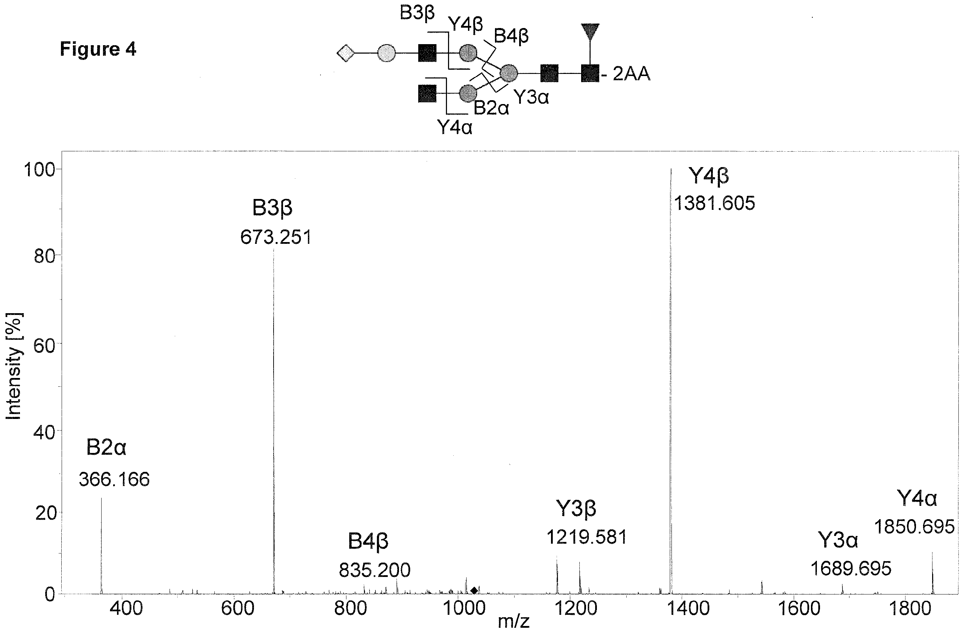

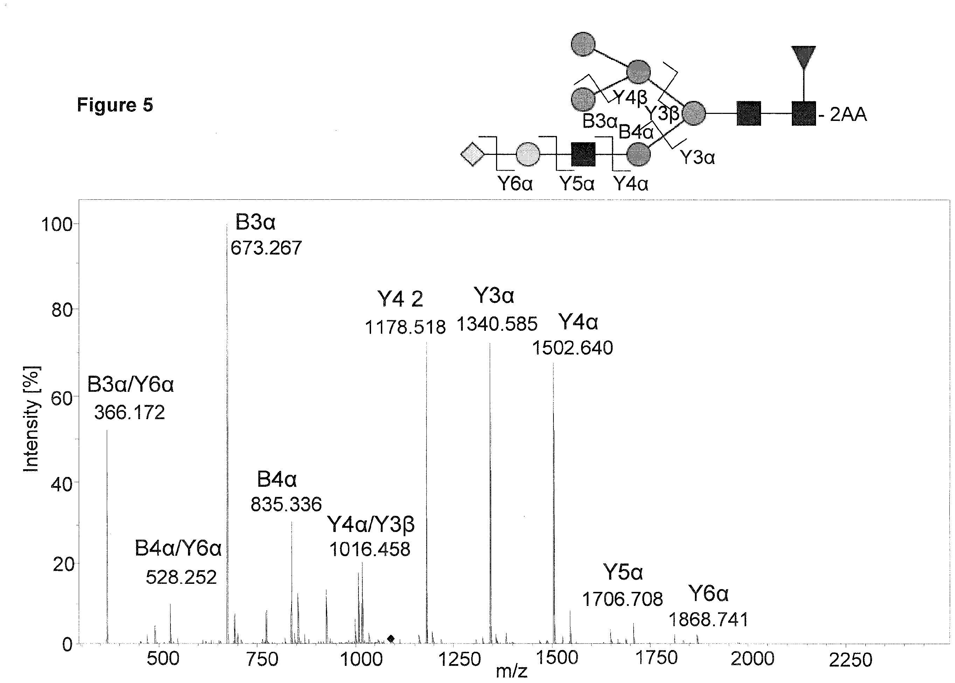

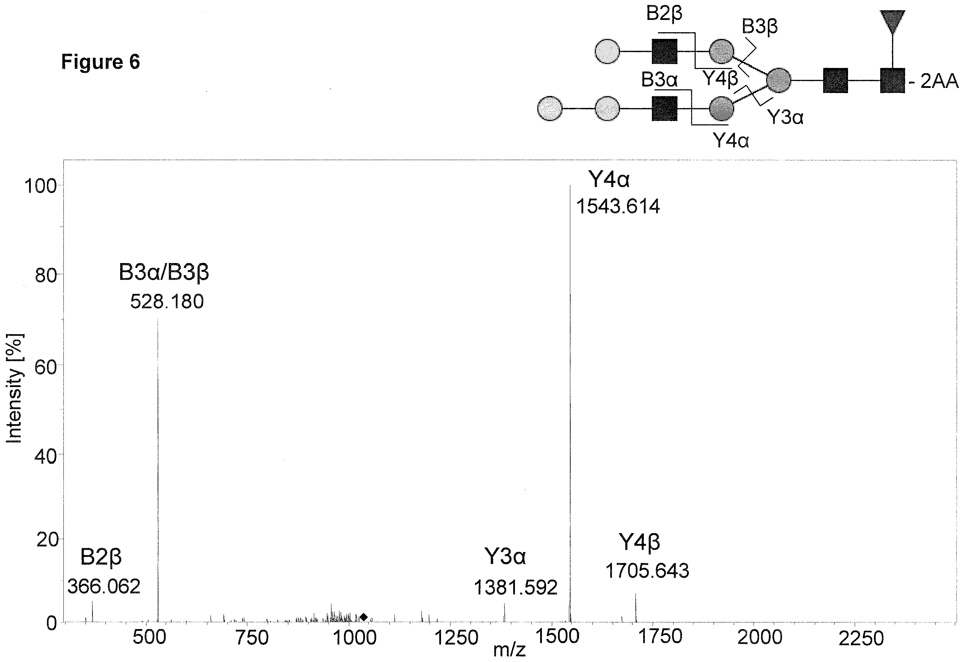

Figure 4 and Figure 5 show the MS² spectra obtained after fragmentation of different N-glycoyl-neuraminic acid containing 2-AA glycans. The spectra were acquired on-line using positive ionization ESI-MS and CID fragmentation. Each of the G1FSG 2-AA glycan fragments (Figure 4) can be explained by the dissociation of a single bond as it is expected using CID in ion-traps. The MS² spectrum at *m*/*z* 1027.9 is dominated by B and Y ions. The sialic acid containing M5G1FSG (Figure 5) shows a slightly different fragmentation pattern. In the MS² spectrum of the [M+2H]²⁺ ion at *m*/*z* 1088.4 only B ions are observed derived from the GlcNAc containing branch, but not from the mannose containing branch of the hybrid structure. The fragments B_{3α}/Y_{6α} with the *m*/*z* 528 and 366 respectively can be explained by the loss of the terminal sialylation. Figure 6 shows the MS² spectrum at *m*/*z* 1035.9 of the rare G3F glycan accounting for less than 0.01% of the glycan pool. B ions are observed for both branches of the glycan since both contain a GlcNAc. This 2-AA glycan co-eluted with the two overlapping and more abundant M3G0F and G2F peaks, but it could be identified and quantified due to on-line MS detection.

### Selectivity of the two developed approaches

The complexity of glycosylation is not only given by the multitude of different N-glycan variants with varying monosaccharide composition. It is also due to the existence of structural isomers with different linkage types. To obtain a glycan-map as comprehensive as possible it is important to separate these isomers.

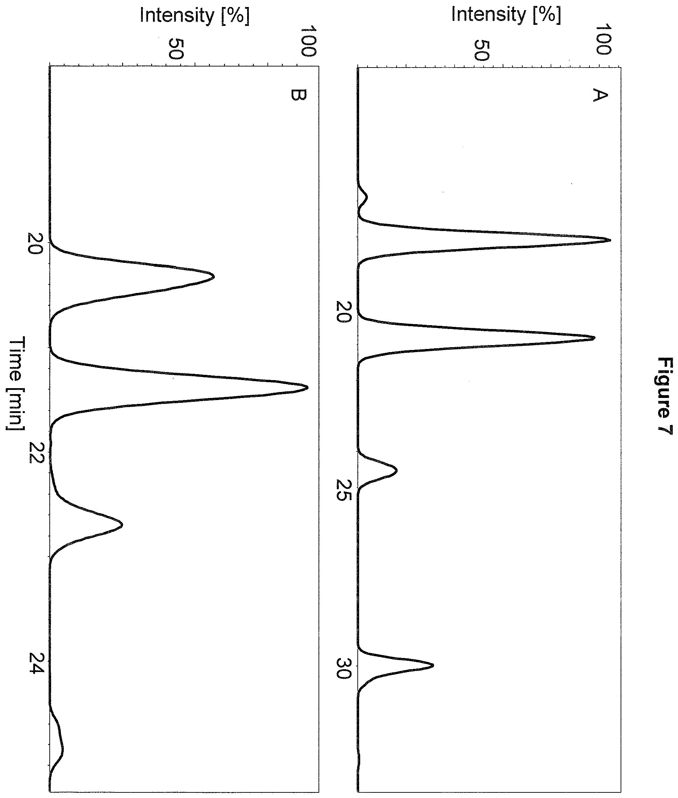

Figure 7 shows the EIC of the M7 isomers of mAb2 for the 2-AB (Figure 7A) as well as for the 2-AA (Figure 7B) labeled glycans. Four different isomers are observed for this high mannose glycan. The linkage could not be deduced with the used reducing end derivatization. The selectivity of the two methods is identical for the high mannose structures only the elution order changed slightly.

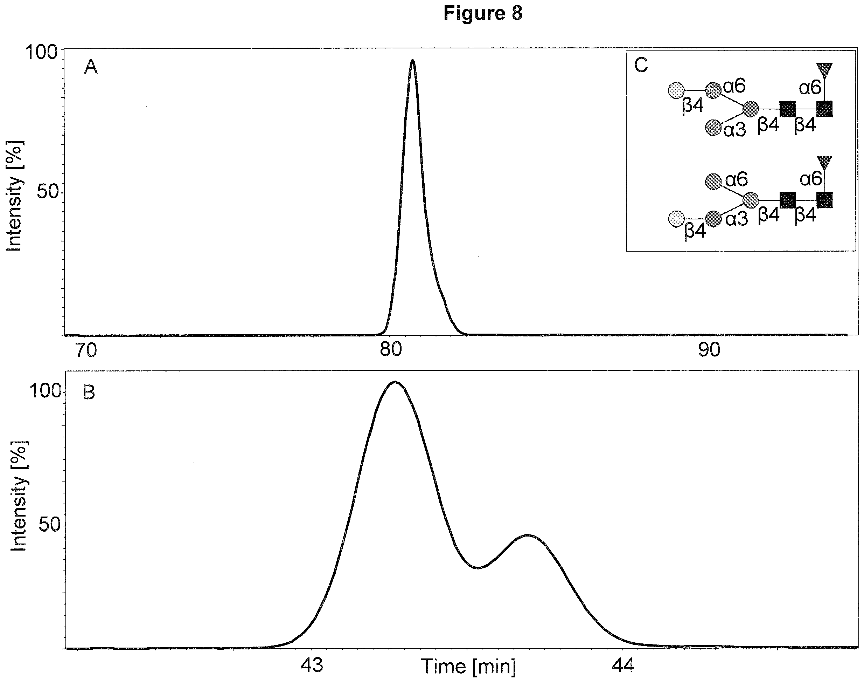

The most abundant structural isomers on mAbs and IgG in general are the G1 F isomers (Figure 8C) with the terminal galactose residue at the α1,3 or the α1,6 branch (G.C. Flynn, X. Chen, Y.D. Liu, B. Shah, Z. Zhang, Naturally occurring glycan forms of human immunoglobulins G1 and G2, Molecular Immunology 47, 2074-82 (2010)). So far separation of these two isomers was only described for normal phase and porous graphitized liquid chromatography (M. Melmer, T. Stangler, A. Premstaller, W. Lindner, Comparison of hydrophilic-interaction, reversed-phase and porous graphitic carbon chromatography for glycan analysis. Journal of Chromatography. A 1218, 118-23 (2011)). In Figure 8 it is clearly shown that the combination of 2-AA as label and the chosen RP chromatography system is capable of separating these isomers whereas the 2-AB RP method is not. Figure 8A shows the EIC of the G1F 2-AB glycan which eluted in one peak as a separation could not be achieved throughout the method development. Figure 8B demonstrates the selectivity of the 2-AA method towards the complex N-glycans. From quantitative data obtained by HILIC chromatography we could deduce that the first, bigger peak is the α1,6 isomer and the second smaller peak corresponds to the α1,3 isomer.

### High sensitivity through high injection volume

The possibility of high injection volumes in RP-LC enables the detection and identification of N-glycan variants which occur only at very low level by fluorescence detection or mass spectrometry. Compared to HILIC where only a few µl of an aqueous sample can be injected this circumstance is of huge advantage as the labeled and purified N-glycans were eluted in 600 µl in the last step of the sample preparation. The sample could be injected without any additional concentration steps. N-glycans accounting for less than 0.01% of the whole mAb N-glycan pool like G3F (shown in Figure 6) could be easily detected and identified by ion-trap MS resulting in a high sensitivity glycan-map of the analyzed mAb.

### Analysis of different glycosylated monoclonal antibodies (mAbs)

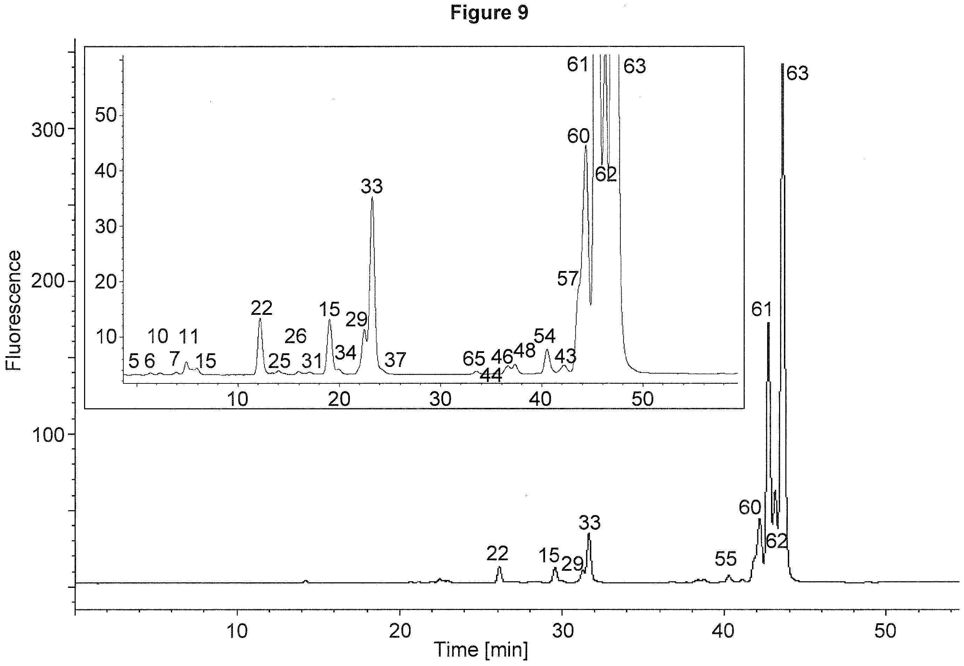

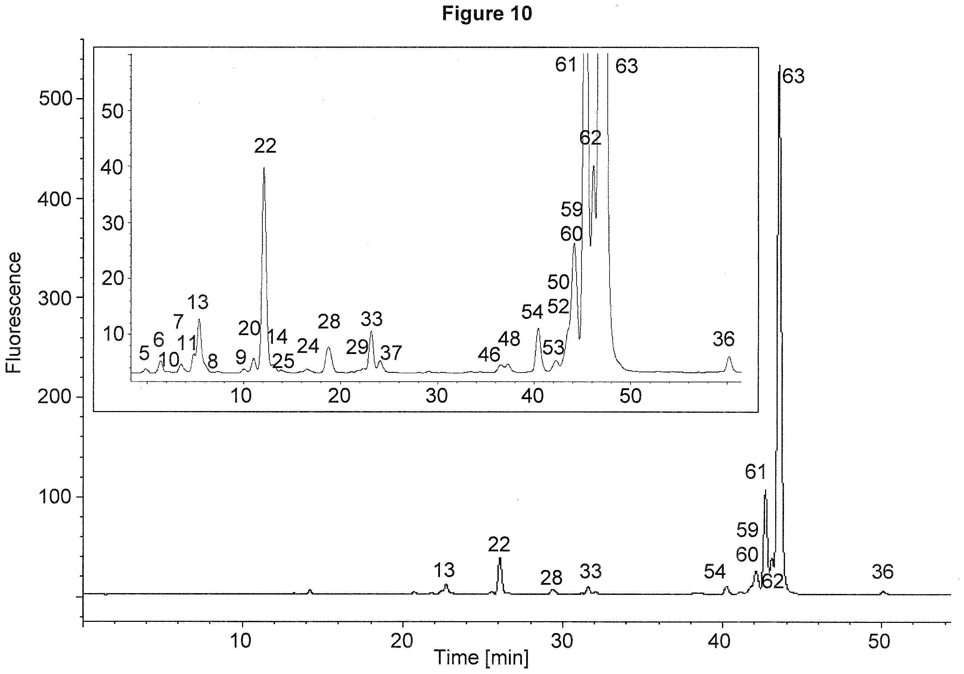

The glycosylation pattern of three different mAbs was analyzed using the developed 2-AA RP LC-MS method to demonstrate the flexibility and versatility of the method for mAb N-glycan characterization. The fluorescence chromatograms are shown in Figures 9-11. These mAbs were chosen due to their different glycan pool. The N-glycosylation pattern of mAb1 (Figure 9) had the lowest amount of high mannose structures, but the highest portion of non-fucosylated complex glycans. Moreover it was the only mAb with both N-acetyl-neuraminic acid and N-glycoyl-neuraminic acid. It comprised 2% high mannose structures, < 1% hybrid structures without core fucose, and 7% complex structures lacking the core fucose. Hybrid and complex N-glycans with α1,6 core fucose accounted for < 1 % and 90% respectively. About 2% of the N-glycans carried a terminal sialic acid. mAb2 (Figure 10) had the highest amount of different oligomannose glycans and carried no sialylation at all. High mannose structures accounted for 7% of all glycans. Non fucosylated hybrid accounted for < 1% and non-fucosylated complex types made 2% of the glycan pool. Hybrid and complex glycoforms with core fucose accounted for 1% and 90%, respectively.

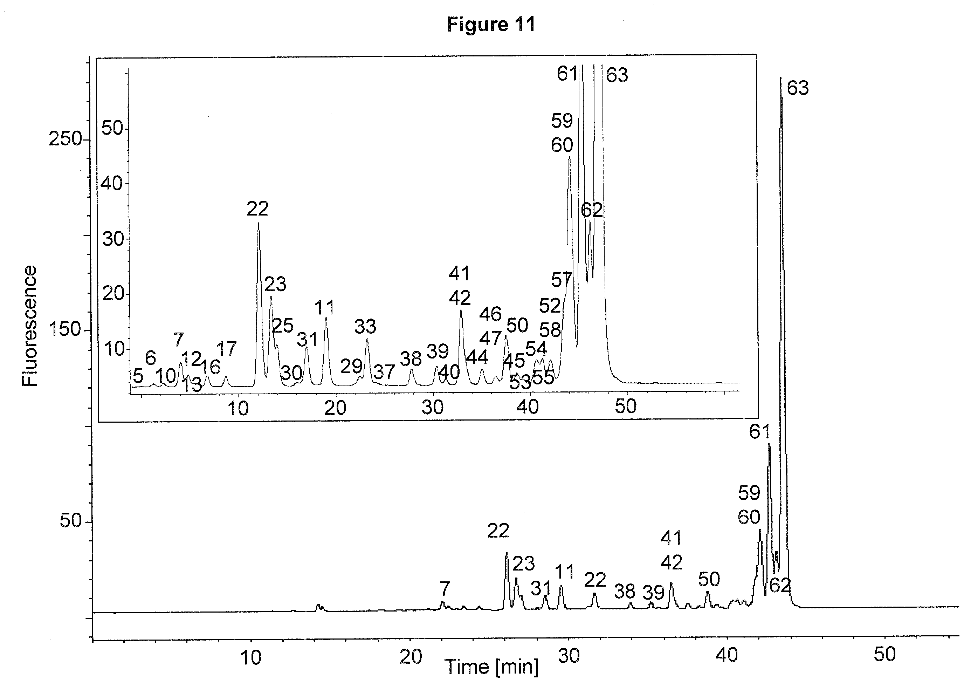

mAb3 (Figure 11) showed the most complex N-glycosylation pattern of the analyzed biopharmaceuticals. It was characterized by a high amount of hybrid structures as well as oligomannose and several sialic acid moieties carrying glycans. The glycosylation comprised 5% oligomannose structures and 5% non-fucosylated hybrid structures. Complex variants accounted for 4% of all glycans. Fucosylated hybrid glycans were present with 5%. Complex fucosylated glycans accounted for 85% and the sialylation level was rather high with 5% for this mAb. The relative glycan composition of the three mAbs was calculated using the peak area of the fluorescence chromatogram of the RP LC runs, for unresolved or co-eluting glycans the EIC was used for integration. The assigned N-glycan structures to each numbered peak are illustrated in Figure 3.

### Conclusions

The developed glycan map platform according to the invention, using RP-HPLC of 2-AB or 2-AA labeled N-glycans, respectively, in combination with fluorescence detection and on-line ion-trap mass spectrometry offers high sensitivity and high resolution for N-glycan analysis. The 2-AA method enables the analysis of neutral and acidic N-glycans with positive ESI MS. Compared to the RP-LC-MS of 2-AB labeled N-glycans more structural isomers can be separated in a shorter analysis time. Especially separation of the highly abundant G1F isomers was achieved. The 2-AA chromatogram of the mAb glycan standard (Figure 2) shows more overlapping peaks or co-eluting peaks respectively which is due to the increased sensitivity and the resulting higher identification rate of the on-line MS detection. The selectivity of the reversed phase chromatography in combination with 2-AA labeling enables separation of various structural isomers from different types of N-glycans and more importantly separates the N-glycans in seven different groups, oligomannose, hybrid and complex without core fucosylation and hybrid and complex with core fucosylation as well as two sialic acid containing groups again with and without the core fucose residue allowing also rapid screening of the glycan composition. The retention of 2-AA on the reversed phase is better compared to 2-AB which may result from a higher hydrophobicity of the protonated carboxyl group of the 2-AA label in the acidic mobile phase. The better retention and separation results in sharper peaks and due to the higher sensitivity of 2-AA in fluorescence detection the analysis of rare glycan species becomes possible. In addition, the focusing of the analyte on the column leads to a more highly concentrated sample entering the ionization chamber of the mass spectrometer and enables efficient ionization and a more sensitive MS detection. Fragmentation of the mostly [M+2H]²⁺ ions by CID produces the mainly occurring B and Y ions providing information about the N-glycan composition. A good ionization of the 2-AA labeled N-glycans was observed in positive ESI MS and even for sialylated structures the MS signal was intensive and on-line MS² data was obtained. Due to the high resolution of the liquid chromatography and the sensitive MS detection, the high complexity of mAb N-glycosylation can be investigated in detail. The high injection volume allows even the detection and quantification of very low abundant N-glycans. The analysis of three mAbs with different glycosylation pattern showed the flexibility of the developed platform for mAb N-glycan characterization. The 2-AA RP LC/MS approach can be used as a robust and orthogonal method for widely established HILIC of 2-AB glycans or MALDI MS of labeled neutral and acidic N-glycans derived from mAbs and other glycoproteins. With the above experimental data, the strength and versatility of RP-LC combined with on-line MS detection for the analysis of N-glycosylation of glycoproteins is demonstrated.

## Claims

1. A method of analyzing the glycans of a glycoprotein, comprising
(i) labeling the glycans with anthranilic acid (2-AA); and
(ii) separating the labeled glycans using reversed-phase liquid chromatography (RP-LC), wherein
(a) the mobile phase used during RP-LC is acidic; and
(b) the reversed-phase liquid chromatography (RP-LC) is performed under conditions under which the carboxyl group of the 2-AA label attached to the glycans is neutral;
and
(iii) subjecting the labeled glycans to positive ionization mass spectrometry (MS) after the separation using RP-LC;
wherein the glycans are neutral or acidic, and
wherein no ion-pairing reagent is used in the mobile phase.

2. The method of claim 1, wherein the glycans are N-glycans of the following types
(i) oligomannose;
(ii) hybrid; or
(iii) complex.

3. The method of claim 2, wherein the N-glycans are G1F isomers, particularly the G1F isomers with 1,3 or 1,6 galactosylation.

4. The method of any one of the preceding claims, wherein the reversed-phase liquid chromatography is
(a) reversed-phase high performance liquid chromatography (RP-HPLC); and/or
(b) done on a reversed-phase liquid chromatography column.

5. The method of any one of the preceding claims, wherein the mobile phase used during RP-LC comprises
(a) formic acid;
(b) formic acid in an amount from 0.1 % to 2.0%; or
(c) formic acid in an amount of 1.0%.

6. The method of any one of the preceding claims, wherein the temperature under which separation is performed is in the range of 40°C to 60°C, or is 50°C.

7. The method of any one of the preceding claims, wherein the flow rate is in a range of 100-500 µl per minute, or is 300 µl per minute.

8. The method of any one of the preceding claims, wherein the mass spectrometry is ion-trap mass spectrometry, particularly positive ionization mass spectrometry.

9. The method of any one of the preceding claims, wherein the mobile phase has a pH
(a) in the range of 1 to 4;
(b) in the range of 1.5 to 3;
(c) in the range of 1.8 to 2.9;
(d) in the range of 1.9 to 2.75
(e) in the range of 2 to 2.7;
(f) in the range of 2.1 to 2.18;
(g) of 2.1; or
(h) of 2.18.

10. The method of any one of claims 1-9, wherein the glycoprotein is
(a) an antibody;
(b) a monoclonal antibody;
(c) a monoclonal antibody of an IgG type; or
(d) a monoclonal antibody of an IgG type selected from IgG1, IgG2, IgG3, or IgG4; or
(e) erythropoietin.

11. Use of 2-AA in a method according to any one of claims 1-10.

## Patentansprüche

1. Verfahren zum Analysieren der Glykane eines Glykoproteins, welches Folgendes umfasst:
(i) Markieren der Glykane mit Anthranilsäure (2-AA); und
(ii) Trennen der markierten Glykane unter Verwendung von Umkehrphasen-Flüssigkeitschromatographie (RP-LC - *reversed-phase liquid chromatography*), wobei
(a) die mobile Phase, die während der RP-LC verwendet wird, sauer ist; und
(b) die Umkehrphasen-Flüssigkeitschromatographie (RP-LC) unter Bedingungen durchgeführt wird, unter welchen die Carboxylgruppe der 2-AA-Markierung, die an die Glykane angehaftet ist, neutral ist; und
(iii) Unterziehen der markierten Glykane einer positiven lonisations-Massenspektrometrie (MS - *mass spectrometry*) nach der Trennung mittels RP-LC;
wobei die Glykane neutral oder sauer sind, und
wobei kein Ionenpaarreagenz in der mobilen Phase verwendet wird.

2. Verfahren nach Anspruch 1, wobei die Glykane N-Glykane des folgenden Typs sind:
(i) Oligomannose;
(ii) Hybrid; oder
(iii) Komplex.

3. Verfahren nach Anspruch 2, wobei die N-Glykane G1F-Isomere sind, insbesondere die G1F-Isomere mit 1,3-oder 1,6-Galaktosylierung.

4. Verfahren nach einem der vorhergehenden Ansprüche, wobei die Umkehrphasen-Flüssigkeitschromatographie:
(a) Umkehrphasen Hochleistungsflüssigkeitschromatographie (RP-HPLC - *reversed-phase high performance liquid chromatography*) ist; und/oder
(b) auf einer Umkehrphasen-Flüssigkeitschromatographie-Säule erfolgt.

5. Verfahren nach einem der vorhergehenden Ansprüche, wobei die mobile Phase, die während der RP-LC verwendet wird, Folgendes umfasst:
(a) Ameisensäure;
(b) Ameisensäure in einer Menge von 0,1 % bis 2,0 %; oder
(c) Ameisensäure in einer Menge von 1,0 %.

6. Verfahren nach einem der vorhergehenden Ansprüche, wobei die Temperatur, unter welcher die Trennung durchgeführt wird, im Bereich von 40 °C bis 60 °C liegt oder 50 °C beträgt.

7. Verfahren nach einem der vorhergehenden Ansprüche, wobei die Durchflussrate in einem Bereich von 100 - 500 µl pro Minute liegt oder 300 µl pro Minute beträgt.

8. Verfahren nach einem der vorhergehenden Ansprüche, wobei die Massenspektrometrie lonenfallen-Massenspektrometrie ist, insbesondere positive lonisierungs-Massenspektrometrie.

9. Verfahren nach einem der vorhergehenden Ansprüche, wobei die mobile Phase einen pH
(a) im Bereich von 1 bis 4;
(b) im Bereich von 1,5 bis 3;
(c) im Bereich von 1,8 bis 2,9;
(d) im Bereich von 1,9 bis 2,75;
(e) im Bereich von 2 bis 2,7;
(f) im Bereich von 2,1 bis 2,18;
(g) von 2,1; oder
(h) von 2,18 aufweist.

10. Verfahren nach einem der Ansprüche 1 - 9, wobei das Glykoprotein Folgendes ist:
(a) ein Antikörper;
(b) ein monoklonaler Antikörper;
(c) ein monoklonaler Antikörper eines IgG-Typs; oder
(d) ein monoklonaler Antikörper eines IgG-Typs ausgewählt aus IgG1, IgG2, IgG3 oder IgG4; oder
(e) Erythropoetin.

11. Verwendung von 2-AA bei einem Verfahren nach einem der Ansprüche 1 - 10.

## Revendications

1. Procédé d'analyse des glycanes d'une glycoprotéine, comprenant :
(i) le marquage des glycanes à l'acide anthranilique (2-AA) ; et
(ii) la séparation des glycanes marqués à l'aide d'une chromatographie liquide en phase inverse (RPLC), où
(a) la phase mobile utilisée au cours de la RPLC est acide ; et
(b) la chromatographie liquide en phase inverse (RPLC) est effectuée dans des conditions dans lesquelles le groupe carboxyle du marquage au 2-AA fixé aux glycanes est neutre ; et
(iii) la soumission des glycanes marqués à une ionisation positive en spectrométrie de masse après la séparation à l'aide de la RPLC ;
où les glycanes sont soit neutres, soit acides, et
où un réactif sans appariement d'ions est utilisé dans la phase mobile.

2. Procédé selon la revendication 1, dans lequel les glycanes sont des N-glycanes des types suivants :
(i) oligomannose ;
(ii) hybride ; ou
(iii) complexe.

3. Procédé selon la revendication 2, dans lequel les N-glycanes sont des isomères G1 F avec une galactosylation en 1,3 ou 1,6.

4. Procédé selon l'une quelconque des revendications précédentes, dans lequel la chromatographie liquide en phase inverse est
(a) une chromatographie liquide haute performance en phase inverse (RP-HPLC) ; et/ou
(b) effectuée sur une chromatographie liquide en phase inverse en colonne.

5. Procédé selon l'une quelconque des revendications précédentes, dans lequel la phase mobile utilisée au cours de la RPLC comprend :
(a) de l'acide formique ;
(b) de l'acide formique à une concentration comprise entre 0,1 % et 2,0 % ; ou
(c) de l'acide formique à une concentration de 1,0 %.

6. Procédé selon l'une quelconque des revendications précédentes, dans lequel la température à laquelle la séparation est effectuée est comprise entre 40 °C et 60 °C, ou est de 50 °C.

7. Procédé selon l'une quelconque des revendications précédentes, dans lequel le débit est compris entre 100 et 500 µl par minute, ou est de 300 µl par minute.

8. Procédé selon l'une quelconque des revendications précédentes, dans lequel la spectrométrie de masse est une spectrométrie de masse à trappe d'ions, notamment une ionisation positive en spectrométrie de masse.

9. Procédé selon l'une quelconque des revendications précédentes, dans lequel la phase mobile possède un pH
(a) compris entre 1 et 4 ;
(b) compris entre 1,5 et 3 ;
(c) compris entre 1,8 et 2,9 ;
(d) compris entre 1,9 et 2,75 ;
(e) compris entre 2 et 2,7 ;
(f) compris entre 2,1 et 2,18 ;
(g) de 2,1 ; ou
(h) de 2,18.

10. Procédé selon l'une quelconque des revendications 1 - 9, dans lequel la glycoprotéine est
(a) un anticorps ;
(b) un anticorps monoclonal ;
(c) un anticorps monoclonal de type IgG ; ou
(d) un anticorps monoclonal de type IgG sélectionné parmi l'IgG1, l'IgG2, l'IgG3 ou l'IgG4 ; ou
(e) l'érythropoïétine.

11. Utilisation du 2-AA dans un procédé selon l'une quelconque des revendications 1 - 10.
